# EUROPÄISCHE PATENTANMELDUNG

(11) **EP 4 763 244 A1**
(43) Veröffentlichungstag der Anmeldung: **24.06.2026**
(21) Anmeldenummer: 25224323.3
(22) Anmeldetag: 17.12.2025
(51) Int. Cl.: A61M 1/36

(54) **GEHÄUSE EINES SICHERHEITSLUFTDETEKTORS EINER DIALYSEMASCHINE**

(30) Priorität: 23.12.2024 DE 102024139597
(71) Anmelder: B. Braun Avitum AG, 34212 Melsungen (DE)
(72) Erfinder: BRESSAU, Ernst, 34323 Malsfeld (DE); HORSCHKE, Sebastian, 37235 Hessisch Lichtenau (DE)
(74) Vertreter: Winter, Brandl - Partnerschaft mbB

(57) **Zusammenfassung**

Offenbart ist ein Gehäuse (1; 101; 201) eines Sicherheitsluftdetektors einer Dialysemaschine, wobei das Gehäuse (1;101; 201) einen Hauptkörper (2; 102) mit einer Blutschlauchaufnahme (6) aufweist, wobei benachbart zur oder in der Blutschlauchaufnahme (6) eine Luftblasensensorik (7) gehäusefest angeordnet ist, und wobei die Blutschlauchaufnahme (6) von einem Deckel (4; 104; 204) zumindest abschnittsweise abdeckbar ist, der mittels zweier Schwenkgelenke (26; 126; 226) um eine Schwenkachse (12) gegenüber dem Hauptkörper (2; 102) schwenkbar ist. Jedes Schwenkgelenk (26; 126; 226) hat einen hauptkörperseitigen Achszapfen (24; 124; 224) und eine deckelseitige Achsaufnahme (28; 128; 228), wobei die Achsaufnahme (28; 128; 228) einstückig und integral mit einem Hauptabschnitt (10) des Deckels (4; 104; 204) gebildet ist. Zwischen einem der Schwenkachse (12) gegenüberliegenden Rand des Hauptabschnitts (10) des Deckels (4; 104; 204) und dem Hauptkörper (2; 102) ist eine Rastvorrichtung mit zumindest einem hauptkörperseitigen Rastelement (16; 116) und mit zumindest einem deckelseitigen Rastelement (14;114) gebildet. Ein Ausweichen der beiden Rastelemente (14, 16; 114; 116) relativ zueinander ist durch eine Elastizität mindestens einer Achsaufnahme (128) oder durch eine Elastizität mindestens eines elastischen Abschnitts (22; 222) des Deckels (4; 104; 204) ermöglicht, die oder der integral und einstückig mit oder an dem Deckel (4; 104; 204) gebildet ist/sind.

## Beschreibung

### Technisches Gebiet

Die vorliegende Offenbarung betrifft ein Gehäuse eines Sicherheitsluftdetektors (SAD) einer Dialysemaschine.

### Hintergrund der Offenbarung

In der Medizin werden Dialysemaschinen zur extrakorporalen Blutbehandlung von Patienten eingesetzt. Bei der Hämodialyse z.B. wird üblicherweise das Doppel-Nadel-Verfahren verwendet. Dabei wird dem Patienten Blut aus dem arteriellen Gefäßzugang entnommen. Die Blutpumpe fördert das Blut kontinuierlich durch das arterielle Blutschlauchsystem zum Dialysator. Der Austausch von Stoffwechselabbauprodukten zwischen dem Blut und der Dialysierflüssigkeit erfolgt über die semipermeable Membran des Dialysators. Danach wird das Blut durch ein venöses Blutschlauchsystem über die venöse Kammer, die als Luftfalle dient, und einen zweiten Gefäßzugang in die Vene des Patienten zurückgegeben.

Dabei wird an dem Schlauch, durch den das Blut zur Vene des Patienten geführt wird, ein Sensor für Luftblasen angeordnet, da diese nicht in den Blutkreislauf des Patienten gelangen dürfen. Derartige Sensoren werden auch Sicherheitsluftdetektoren (SAD) genannt.

### Stand der Technik

Aus dem Stand der Technik ist es bekannt an dem Gehäuse einer Dialysemaschine ein Gehäuse eines Sicherheitsluftdetektors zu befestigen bzw. anzuordnen/ vorzusehen, das auch als Sensorgehäuse bezeichnet werden kann. In einen Hauptkörper des Sensorgehäuses wird ein Blutschlauch eingelegt, und von einem Deckel des Sensorgehäuses verschlossen. Dabei ist der Deckel über ein Schwenkgelenk an dem Hauptköper des Sensorgehäuses angelenkt und kann entsprechend auf- und zugeklappt werden.

Im Zusammenhang mit Dialysemaschinen offenbaren die Druckschriften EP 2 679 752 B1, JP 4812101 B2 und US 3,963,023 A flankierenden Stand der Technik. Jede Druckschrift zeigt ein Gehäuse einer Blutpumpe einer Dialysemaschine, deren schwenkbarer bzw. klappbarer Deckel mit einer Feder gespannt ist. Genauer gesagt haben die Deckel im Endergebnis Torsionsfedern, auch wenn diese durch eine Schraubenfeder und einen deckelseitigen unrunden Körper des Schwenkgelenks gebildet sind. Damit werden torsionsgefedert schwenkende Öffnungs- und Schließbewegungen des Deckels erzeugt.

Aus hausinternem Stand der Technik, der z.B. an der Dialysemaschine "Dialog iQ" der Anmelderin vorgesehen ist, ist weiterhin ein Gehäuse eines Sicherheitsluftdetektors bekannt, dessen Deckel über zwei topfförmige Achsaufnahmen an zwei Achszapfen gelagert sind. Die beiden Achsaufnahmen und die beiden Achszapfen sind konzentrisch und bilden so eine geometrische Schwenkachse des Deckels. Der Deckel weist zum Hintergreifen zweier gehäuseseitiger Rastnasen zwei entsprechende Rasthaken auf. Am Anfang einer Öffnungsbewegung und am Ende einer Schließbewegung ist eine elastische Ausweichbewegung nötig, damit die Rasthaken an den Rastnasen vorbeibewegt werden können. Bei dem genannten Stand der Technik wird diese Ausweichbewegung dadurch ermöglicht, dass getrennt von einem Hauptkörper des Gehäuses ein vergleichsweise großes stirnseitiges Gehäuseteil vorgesehen ist, an dem die beiden gehäuseseitigen Rastnasen gebildet sind, und das über eine Feder nach außen in Richtung zu den beiden deckelseitigen Rasthaken gespannt ist. Dieses Gehäuseteil kann am Anfang der Öffnungsbewegung und am Ende der Schließbewegung gegen die Kraft der Feder zum Hauptkörper des Gehäuses bewegt (gedrückt) werden und damit den Rasthaken ausweichen.

Es hat sich an derartigen Gehäusen für Sicherheitsluftdetektoren von Dialysemaschinen jedoch gezeigt, dass eine komplette keimfreie Reinigung des Gehäuses wegen der Mehrteiligkeit des Hauptkörpers und wegen der durch das stirnseitige Gehäuseteil verdeckten Feder schwierig ist.

### Kurzbeschreibung der Offenbarung

Aufgabe der vorliegenden Offenbarung ist es, ein Gehäuse für einen Sicherheitsluftdetektor einer Dialysemaschine zu schaffen, dessen (vollumfängliche) Reinigung erleichtert ist. Weiterhin soll auch die Teileanzahl reduziert und damit die Montage des Gehäuses bzw. des Sicherheitsluftdetektors erleichtert werden.

Diese Aufgabe wird mit der Merkmalskombination des Anspruchs 1 gelöst.

Das Gehäuse eines Sicherheitsluftdetektors einer Dialysemaschine gemäß der Offenbarung hat einen Hauptkörper mit einer z.B. rinnenförmigen Blutschlauchaufnahme, in die ein Blutschlauch eingelegt werden kann. Benachbart zum Blutschlauch ist eine Luftblasensensorik gehäusefest angeordnet, z.B. sind an radial gegenüberliegenden Seiten der Blutschlauchaufnahme verschiedene Komponenten der Luftblasensensorik angeordnet. Die Blutschlauchaufnahme ist von einem Deckel zumindest abschnittsweise abdeckbar oder verschließbar, der vorzugsweise auch den Blutschlauch in die Blutschlauchaufnahme spannt. Der Deckel kann darüber hinaus gehend auch den gesamten Hauptkörper abdecken. Der Deckel ist um eine Schwenkachse, die sich vorzugsweise parallel zur Blutschlauchaufnahme erstreckt, gegenüber dem Hauptkörper schwenkbar. Daher kann der Deckel auch als Klappe bezeichnet werden. Dazu sind zwischen dem Hauptkörper und dem Deckel zwei Schwenkgelenke vorgesehen, wobei jedes Schwenkgelenk einen hauptkörperseitigen Achszapfen und eine deckelseitige Achsaufnahme hat. Die Achsaufnahme ist integral und einstückig (z.B. als Kunststoff-Spritzgussteil) mit dem Rest, insbesondere mit einem Hauptabschnitt des Deckels gebildet. Zwischen einem der Schwenkachse gegenüberliegenden Rand des Hauptabschnitts des Deckels und dem Hauptkörper des Gehäuses ist eine Rastvorrichtung mit zumindest einem hauptkörperseitigen Rastelement und mit zumindest einem deckelseitigen Rastelement gebildet. Die Rastelemente können je nach Ausprägung auch als Rasthaken oder Rastnasen bezeichnet werden. Offenbarungsgemäß wird das beim Öffnen oder Schließen des Deckels nötige Ausweichen der beiden Rastelemente relativ zueinander durch eine Elastizität mindestens einer der beiden Achsaufnahmen, vorzugsweise beider Achsaufnahmen, oder durch eine Elastizität mindestens eines anderen elastischen Abschnitts, vorzugsweise zweier anderer elastischer Abschnitte, des Deckels ermöglicht. Der oder die elastische(n) Abschnitt(e) können auch als elastische Bereiche bezeichnet werden. Im Falle des von der Achsaufnahme abweichenden elastischen Abschnitts ist auch dieser integral und einstückig (z.B. als Kunststoff-Spritzgussteil/ eines Abschnitts desselben) mit oder an dem Deckel gebildet. Damit wird offenbarungsgemäß die nötige Ausweichbewegung in Form von translatorischer Verschiebung des Deckels gegenüber dem Hauptkörper ermöglicht. Damit ist ein Gehäuse für einen Sicherheitsluftdetektor einer Dialysemaschine geschaffen, dessen Reinigung erleichtert ist, denn es entfällt das extra Bauteil des Gehäuses, das gemäß dem Stand der Technik vorgesehen ist. Auch die von diesem extra Bauteil verdeckte Feder, die zum elastischen Ausweichen der Rastelemente des Standes der Technik vorgesehen ist, entfällt. Damit ist die Teileanzahl des Gehäuses um zwei Teile reduziert und damit die Reinigung und die Montage erleichtert.

Die elastische(n) Achsaufnahme(n) und/oder der mindestens eine elastische Abschnitt kann/können von einem Werkstoff (insbesondere Kunststoff) gebildet sein, der elastischer und/oder weicher als der Werkstoff (insbesondere Kunststoff) der übrigen Abschnitte des Deckels ist. Im Falle der Lösung aus Kunststoff ist der gesamte Deckel vorzugsweise ein Kunststoffspritzgussteil, und der elastischere und/oder weichere Kunststoff ist integral mit dem übrigen Kunststoff gespritzt, beispielsweise im Zwei-Komponenten-Spritzguss.

Im Falle der Lösung mit dem mindestens einen elastischen Abschnitt kann dieser auch körperlich dünner oder schmaler als ein benachbarter Abschnitt oder als benachbarte Abschnitte des Deckels sein. So kann z.B. ein Verbindungabschnitt, der zwischen der Achsaufnahme und dem Hauptabschnitt des Deckels angeordnet ist, vergleichsweise dünn und/oder schmal ausgelegt sein, so dass auch bei gleichem (unveränderten) Werkstoff (z.B. Kunststoff) somit der elastische Abschnitt gebildet ist. Bei Belastung durch das Ausweichen der Rastelemente tritt dann am Verbindungsabschnitt die nötige Elastizität in Form einer elastischen Biegung auf. Der Verbindungsabschnitt kann alternativ auch ein elastischeres/ weicheres Material aufweisen. Weiter alternativ kann auch der Hauptabschnitt des Deckels, gegebenenfalls sogar im Bereich des deckelseitigen Rastelements, den elastischen Abschnitt aufweisen.

Bei einer besonders bevorzugten Ausgestaltung der Achsaufnahme hat diese an einer der Rastvorrichtung zugewandten Seite eine gekrümmte Anlage, die ein Gleitlager für den Achszapfen bildet. Die gekrümmte Anlage hat (im Schnitt betrachtet) eine Kreisbogenform (maximal Halbkreisbogenform oder kürzer).

Vorzugsweise ist eine Spielpassung zwischen der gekrümmten Anlage und einem Außenmantel des Achszapfens gebildet. In anderen Worten ausgedrückt haben die gekrümmte Anlage und der Außenmantel des Achszapfens einen etwa gleichen Radius, so dass der Achszapfen passgenau in der gekrümmten Anlage aufgenommen ist. Dann kann sich die gekrümmte Anlage reibungsarm und präzise geführt um den Achszapfen herumbewegen.

Die gekrümmte Anlage kann zu einem vollumfänglichen Hülsenabschnitt (Zylinderabschnitt) weitergebildet sein. In anderen Worten ausgedrückt kann die Achsaufnahme einen um den Achszapfen umlaufenden Hülsenabschnitt (Zylinderabschnitt) haben, wobei die gekrümmte Anlage ein Abschnitt des Hülsenabschnitts ist.

Um die nötige Ausweichbewegung durch translatorische Verschiebung des Deckels gegenüber dem Hauptkörper zu ermöglichen, ist der Hülsenabschnitt vorzugsweise länglich. Der Hülsenabschnitt kann also (im Schnitt betrachtet) oval sein.

Bei einer bevorzugten Ausgestaltung hat die Achsaufnahme einen stirnseitigen Abschlussdeckel, der im Wesentlichen senkrecht zur Schwenkachse ist, womit die Achsaufnahme topfartig ist. Daher kann der Abschlussdeckel auch als Boden der topfartigen Achsaufnahme ausgestaltet sein. Damit kann die axiale Verschiebbarkeit des Deckels gegenüber dem Hauptkörper entlang der Schwenkachse begrenzt oder ganz vermieden werden. Im Fall von zwei jeweiligen Abschlussdeckeln an beiden Achsaufnahmen ist der Deckel des Gehäuses axial am Hauptkörper festgelegt und es ist sichergestellt, dass die Rastelemente sich beim Schließen des Deckels (paarweise) treffen.

Bei einem ersten Ausführungsbeispiel ist der elastische Abschnitt ein eigeständiges zusätzliches körperliches Bauteil, das an der Achsaufnahme gebildet und radial gegen einen Außenmantel des Achszapfens gespannt ist. Dabei liegt der elastische Abschnitt in einem von der Rastvorrichtung abgewandten Umfangsbereich des Außenmantels an diesem an.

Der elastische Abschnitt ist dabei z.B. von einer Federzunge oder Federlasche gebildet, die im Innern der Achsaufnahme, insbesondere am Innenumfang des Hülsenabschnitts, befestigt ist und sich zumindest abschnittsweise tangential zum Achszapfen erstreckt. Damit die Federzunge oder Federlasche nicht vom Achszapfen abrutscht, kann sie an ihrer dem Achszapfen zugewandten Seite eine konkave Krümmung aufweisen, in die der Achszapfen eintaucht.

Bei einem zweiten Ausführungsbeispiel ist das Ausweichen der beiden Rastelemente relativ zueinander durch die Elastizität der beiden Achsaufnahmen ermöglicht.

Zusätzlich oder alternativ kann jede Achsaufnahme über einen Verbindungsabschnitt mit dem Hauptabschnitt des Deckels einstückig und integral (z.B. als Kunststoff-Spritzgussteil) verbunden sein, und das Ausweichen der beiden Rastelemente relativ zueinander ist durch die Elastizität der beiden Verbindungabschnitte ermöglicht.

Gemäß einem dritten Ausführungsbeispiel kann auch der Hauptabschnitt des Deckels, gegebenenfalls sogar im Bereich des deckelseitigen Rastelements, die Elastizität aufweisen.

Bei einer konkreten Weiterbildung des zweiten Ausführungsbeispiels ist im Innern der Achsaufnahme beabstandet zur Schwenkachse eine deckelseitige Aktivierungsschräge gebildet, während am Achszapfen ebenfalls beabstandet zur Schwenkachse eine hauptkörperseitige Aktivierungsschräge gebildet ist. Beide Aktivierungsschrägen sind an einer gemeinsamen von der Rastvorrichtung abgewandten Seite der Schwenkachse angeordnet. Beide Aktivierungsschrägen sind in gleicher Richtung und vorzugsweise mit etwa gleichem Winkel schräg zur Schwenkachse angeordnet. Beim Ausweichen der Rastelemente, also bei einer Bewegung des Deckels relativ zum Hauptkörper, wird eine zunehmende Überdeckung der beiden Aktivierungsschrägen erzeugt, wodurch die beiden Achsaufnahmen entlang der Schwenkachse auseinandergedrängt werden.

Wenn der Achszapfen eine vom Hauptkörper abgewandte (z.B. in einer Projektion kreisrunde) Stirnfläche hat, kann die hauptkörperseitige Aktivierungsschräge vorrichtungstechnisch einfach ein von der Rastvorrichtung abgewandter Oberflächenabschnitt dieser Stirnfläche sein.

Im Falle der Stirnfläche am Achszapfen kann ein anderer Oberflächenabschnitt dieser Stirnfläche eine Einführschräge bilden, die schräg zur Schwenkachse und schräg zur hauptkörperseitigen Aktivierungsschräge angeordnet ist. Diese Einführschräge kann eine Montagehilfe beim Aufsetzen des Deckels auf den Hauptkörper, also beim Verbinden der Achsaufnahmen mit den Achszapfen sein.

Die deckelseitige Aktivierungsschräge kann an einem Vorsprung oder an einer Rippe gebildet sein, der oder die im Innern der Achsaufnahme an einer von der Rastvorrichtung abgewandten Seite der Schwenkachse angeordnet ist. Der Vorsprung oder die Rippe erstreckt sich radial zur Schwenkachse nach innen.

### Kurzbeschreibung der Figuren

Figur 1 ist ein Gehäuse gemäß einem ersten Ausführungsbeispiel der vorliegenden Offenbarung in einer geschnittenen Seitenansicht.
Figur 2 ist ein Hauptkörper des Gehäuses aus Figur 1 in einer Seitenansicht.
Figur 3 ist ein Deckel des Gehäuses aus Figur 1 in einer geschnittenen Seitenansicht.
Figur 4 ist ein Gehäuse gemäß einem zweiten Ausführungsbeispiel der vorliegenden Offenbarung in einer durchscheinenden Seitenansicht.
Figur 5 ist ein Schwenkgelenk des Gehäuses aus Figur 4 in einer Draufsicht.
Figur 6 ist ein Gehäuse gemäß einem dritten Ausführungsbeispiel der vorliegenden Offenbarung in einer durchscheinenden Seitenansicht.

### Beschreibung der Ausführungsbeispiele

Nachstehend werden drei Ausführungsbeispiele der vorliegenden Offenbarung auf der Basis der zugehörigen Figuren beschrieben.

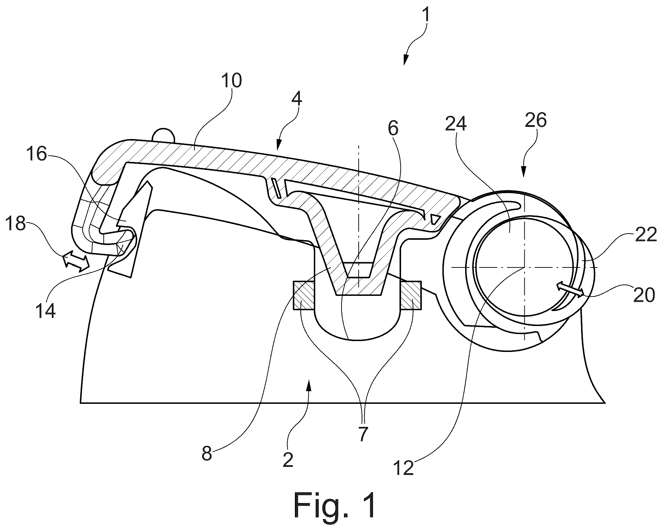

Figur 1 ist ein Gehäuse 1 eines Sicherheitsluftdetektors gemäß einem ersten Ausführungsbeispiel der vorliegenden Offenbarung in einer geschnittenen Seitenansicht. Ein (nicht gezeigter) Blutschlauch wird (in Figur 1 senkrecht zur Zeichenebene) in eine Blutschlauchaufnahme 6 eingelegt. Seitlich an eine Blutschlauchaufnahme 6 ist eine Luftblasensensorik 7 angeordnet, mit der eventuell vorhandene Luftblasen des im Blutschlauch geführten Bluts erkannt werden. Die Blutschlauchaufnahme 6 ist etwa rinnenförmig in einem Hauptkörper 2 des Gehäuses 1 gebildet.

Um eine optimale Wirkverbindung zwischen dem Blut in dem Blutschlauch einerseits und der Sensorik der Blutschlauchaufnahme 6 andererseits herzustellen, wird der Blutschlauch durch einen Niederhalter 8 in die Blutschlauchaufnahme 6 hineingedrückt und dort gehalten. Der Niederhalter 8 ist integral an einem Hauptabschnitt 10 eines Deckels 4 des Gehäuses 1 gebildet. Der Deckel 4 ist um eine Schwenkachse 12 gegenüber dem Hauptkörper 2 des Gehäuses 1 schwenkbar. Die Schwenkachse 12 ist (auch bei der weiter unten beschriebenen Ausweichbewegung 18) ruhend und unbeweglich gegenüber dem Hauptkörper 2 und erstreckt sich parallel zur Blutschlauchaufnahme 6.

Die Schwenkachse 12 wird definiert von zwei kreiszylindrischen Achszapfen 24, die sich ausgehend vom Hauptköper 2 entlang der Schwenkachse 12 erstrecken. Die beiden Achszapfen 24 sind jeweils ein Teil von entsprechenden Schwenkgelenken 26, wobei in Figur 1 ist nur eines dieser Schwenkgelenke 26 zu sehen ist. Die beiden Achszapfen 24 können separat voneinander ausgebildet sein, können jedoch auch Abschnitte einer durchgehenden Achse sein. Es ist auch denkbar, dass die Achszapfen 24 integrale Abschnitte des Hauptkörpers 2 sind.

Figur 1 zeigt den Deckel 4 in seiner geschlossenen Stellung. In dieser Stellung hintergreift ein deckelseitiges Rastelement 14 ein hauptkörperseitiges Rastelement 16. Beim gezeigten Ausführungsbeispiel ist das deckelseitige Rastelement 14 als Rasthaken ausgebildet, während das hauptkörperseitiges Rastelement 16 demgegenüber etwas schwächer als Rastnase ausgeprägt ist.

Im Gegensatz zum oben genannten hausinternem Stand der Technik ist das hauptkörperseitige Rastelement 16 nicht elastisch beweglich gegenüber dem Hauptkörper 2, sondern fest an diesem angeordnet oder gebildet, vorzugsweise integral im Kunststoffspritzgussverfahren gebildet.

Beim Öffnen und beim Schließen des Deckels 4 gegenüber dem Hauptkörper 2 führt daher das deckelseitige Rastelement 14 eine elastisch belastete Ausweichbewegung 18 relativ zum hauptkörperseitigen Rastelement 16 gemäß dem Doppelpfeil 18 aus.

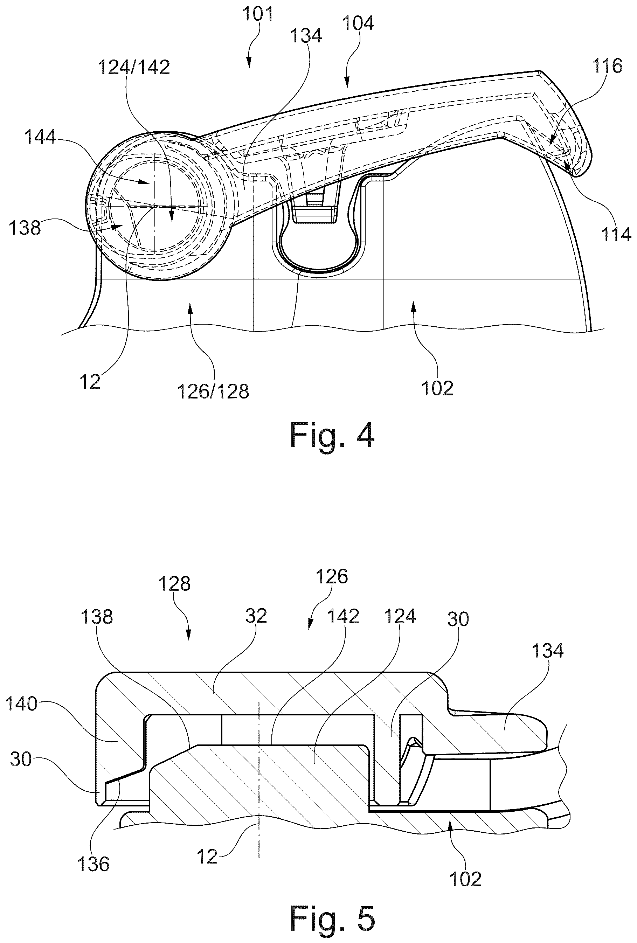

Dazu ist bei beiden gezeigten Ausführungsbeispielen des Gehäuses 1; 101 der gesamte Hauptabschnitt 10 des Deckels 4 mit dem deckelseitigen Rastelement 14 (in Figur 1 nach links und in Figur 4 nach rechts) elastisch translatorisch bewegbar.

Diese elastische Ausweichbewegung 18 wird beim ersten Ausführungsbeispiel gemäß Figur 1 entsprechend dem Doppelpfeil 20 durch eine Federzunge 22 erzeugt bzw. erlaubt, die einstückig und integral im Kunststoff-Spritzgussverfahren mit dem Deckel 4 gebildet ist, und die stets an einem Außenmantel des Achszapfens 24 anliegt, der fest und ruhend an dem Hauptkörper 2 gebildet ist. Bei der Ausweichbewegung 18 wird die Federzunge 22 von dem Achszapfen 24 radial von der Schwenkachse 12 weg nach außen gedrängt. Danach drängt die Federzunge 22 den Deckel 4 wieder zurück (in Figur 1 nach rechts), und damit kommt das deckelseitige Rastelement 14 in den in Figur 1 gezeigten Rasteingriff mit dem hauptkörperseitigen Rastelement 16.

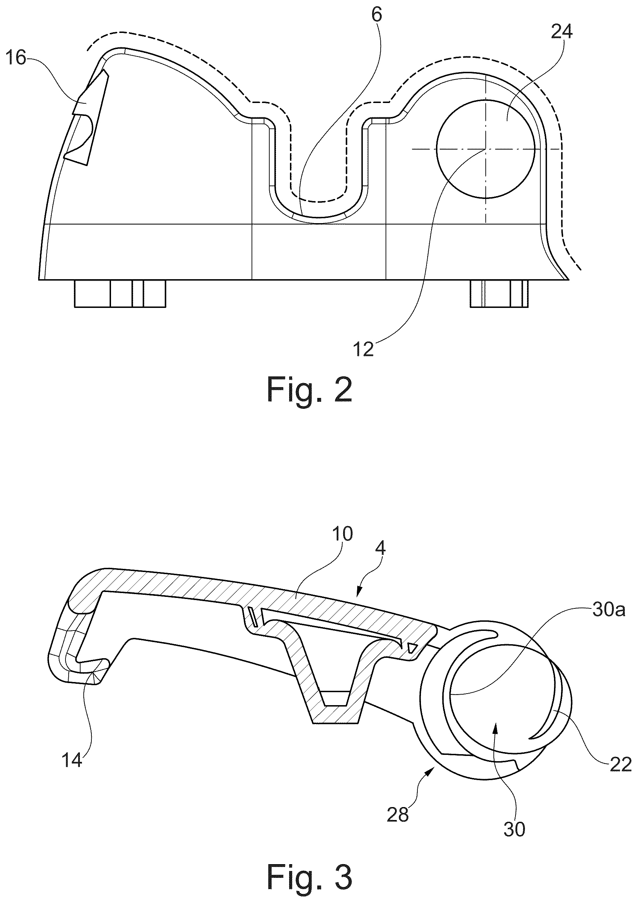

Figur 2 ist der Hauptkörper 2 des Gehäuses 1 aus Figur 1 in einer Seitenansicht. Dabei wurde der Deckel 4 weggelassen, um den Teil des in Figur 1 komplett gezeigten Schwenkgelenks 26 zu zeigen, der an dem Hauptkörper 2 gebildet ist. Dabei handelt es sich lediglich um den kreiszylindrischen Achszapfen 24, wobei die beiden Achszapfen 24 durch ihre zusammenfallenden Mittelachsen die Schwenkachse 12 definieren.

Figur 3 ist der Deckel 4 des Gehäuses 1 aus Figur 1 in einer geschnittenen Seitenansicht. Dabei wurde der Hauptkörper 2 weggelassen, um die Teile des Schwenkgelenks 26 zu zeigen, die an dem Deckel 4 gebildet sind. Diese sind eine topfartige Achsaufnahme 28, die im Wesentlichen aus einem ovalen zylindrischen Hülsenabschnitt 30 und einem Abschlussdeckel gebildet ist, der auch als Boden der topfartigen Achsaufnahme 28 angesehen werden kann. Da die Schnittebene in Figur 3 durch den Hülsenabschnitt 30 verläuft, ist der Abschlussdeckel nicht zu sehen.

Der Hülsenabschnitt 30 hat eine ovale Form, wobei die längere Erstreckung etwa parallel zum Hauptabschnitt 10 des Deckels 4 verläuft, während die kürzere Erstreckung senkrecht dazu verläuft. Die kürzere Erstreckung des ovalen Hülsenabschnitts 30 entspricht etwa dem Durchmesser des in Figur 2 gezeigten Achszapfens 24, die längere Erstreckung ist demgegenüber derart verlängert, dass mindestens die in Figur 1 gezeigte Ausweichbewegung 18 möglich ist.

Im Innern des Hülsenabschnitts 30 der Achsaufnahme 28 des Deckels 4 erstreckt sich außermittig und damit entfernt von der in Figur 2 gezeigten Schwenkachse 12 die (bereits mit Bezug zu Figur 1 genannte) Federzunge 22 aus Kunststoff. Die Federzunge 22 ist einseitig (in Figur 3 oben) einstückig und integral mit dem Hülsenabschnitt 30 und damit mit dem gesamten Deckel 4 verbunden. Weiterhin hat sie (in Figur 3 unten einen freien Endabschnitt. Damit kann sie radial zur Figur 2 gezeigten Schwenkachse 12 und zum Achszapfen 24 ausweichen, wenn der Deckel 4 zur Betätigung der Rastvorrichtung bewegt wird.

In einer Normalposition des Deckels 4 gegenüber dem Hauptkörper 2, in der auch der größte Teil der Schwenkbewegung des Deckels 4 gegenüber dem Hauptkörper 2 erfolgt, wird der Achszapfen 12 von der Federzunge 22 in Anlage mit einer kreisbogenförmig gekrümmten Anlage 30a gehalten, deren Radius demjenigen des Achszapfens 12 entspricht. Die gekrümmte Anlage 30 bildet einen Teil des ovalen Hülsenabschnitts 30.

Während der Ausweichbewegung des Rastelements 14 gegenüber dem Rastelement 16 hebt der Achszapfen 24 übergangsweise von der gekrümmten Anlage 30a ab.

Bei von dem ersten Ausführungsbeispiel gemäß den Figuren 1 bis 3 abweichenden Ausführungsbeispielen kann es reichen, dass lediglich die kreisbogenförmig gekrümmte Anlage 30a und demgegenüber auf der anderen Seite der Schwenkachse 12 die Federzunge 22 vorgesehen sind, so dass kein vollumfänglich geschlossener ovaler Hülsenabschnitt vorgesehen ist.

Figur 4 ist das Gehäuse 101 gemäß dem zweiten Ausführungsbeispiel der vorliegenden Offenbarung in einer durchscheinenden Seitenansicht. Dabei sind abgesehen von einer aneinander angeglichenen Ausprägung bzw. Ausgestaltung der beiden Rastelemente 114, 116 insbesondere die Erzeugung der Elastizität und die Schwenkgelenke 126 von denjenigen des ersten Ausführungsbeispiels abweichend ausgeführt.

Beim zweiten Ausführungsbeispiel wird die nötige Elastizität zur Ermöglichung der Ausweichbewegung des Rastelements 114 relativ zum Rastelement 116 durch die beiden topfartigen Achsaufnahmen 128 und/oder durch deren jeweilige Verbindungsabschnitte 134 zum Hauptabschnitt 10 des Deckels 4 erzeugt. Dabei werden bei der Ausweichbewegung die beiden Achsaufnahmen 128 entlang der Schwenkachse 12 voneinander weg gebogen oder gekrümmt, es wird also ihr Abstand vergrößert.

Figur 5 ist das Schwenkgelenk 126 des Gehäuses 101 aus Figur 4 in einer geschnittenen Draufsicht. Beim Ausführungsbeispiel gemäß den Figuren 4 und 5 wird also der Abstand der beiden Achsaufnahmen 128 zueinander entlang der Schwenkachse 12 vergrößert, wobei in Figur 5 nur eine Achsaufnahme 128 gezeigt ist. Im zweiten Teil der Ausweichbewegung wird der Abstand der beiden Achsaufnahmen 128 zueinander entlang der Schwenkachse 12 wieder in die Ausgangslage verkleinert.

Gemäß Figur 5 sind an der der Rastvorrichtung gegenüberliegenden (linken) Seite der Schwenkachse 12 zwei Aktivierungsschrägen 136, 138 vorgesehen, die sich in der in Figur 5 gezeigten Normalposition nicht überdecken.

Während der Ausweichbewegung des Rastelements 114 relativ zum Rastelement 116 wird die Achsaufnahme 128 (in Figur 5 nach rechts) bewegt, sodass die beiden Aktivierungsschrägen 136, 138 in Anlage aneinander kommen und übereinander geschoben werden und sich dabei die Achsaufnahme 128 entlang der Schwenkachse 12 (in Figur 5 nach oben) elastisch biegt.

Beide Aktivierungsschrägen 136, 138 sind in gleicher Richtung und mit etwa gleichem Winkel schräg zur Schwenkachse 12 angeordnet.

Die deckelseitige Aktivierungsschräge 136 ist an einer Rippe 140 gebildet, die im Eckbereich zwischen dem Hülsenabschnitt 30 und dem Abschlussdeckel 32 gebildet ist, während die hauptkörperseitige Aktivierungsschräge 138 (als Abschrägung) an einer Stirnfläche 142 des Achszapfens 124 gebildet ist.

In Figur 4 ist wegen der durchscheinenden Darstellung des Abschlussdeckels 32 die gesamte Stirnfläche 142 des Achszapfens 124 zu erkennen. Sie hat abgesehen von der oben genannten hauptkörperseitigen Aktivierungsschräge 138 noch eine Einführschräge 144, die als Montagehilfe bei dem erstmaligen Aufsetzen der Achsaufnahmen 128 des Deckels 104 auf die Achszapfen 124 des Hauptkörpers 102 dienen, da auch bei der Montage die Elastizität der Achsaufnahmen 128 und/oder der Verbindungsabschnitte 134 genutzt wird.

In Figur 4 ist wegen der durchscheinenden Darstellung der Achsaufnahme 128 auch die Rippe 140 zu erkennen, an der die deckelseitige Aktivierungsschräge 136 gebildet ist.

Gemäß einer Alternative der zweiten Ausführungsform kann auch lediglich der elastische Verbindungsabschnitt 134 die gewünschte Ausweichbewegung ermöglichen, wobei die gewünschte Elastizität durch eine Materialelastizität und/oder durch ein geeignetes elastisches Design des Deckels 104 bereitgestellt werden kann.

Es kann auch ein anderer elastischer Abschnitt des Deckels die gewünschte Ausweichbewegung ermöglichen, wobei die gewünschte Elastizität durch eine Materialelastizität und/oder durch ein geeignetes elastisches Design z.B. Dünnwandigkeit des Deckels bereitgestellt werden kann.

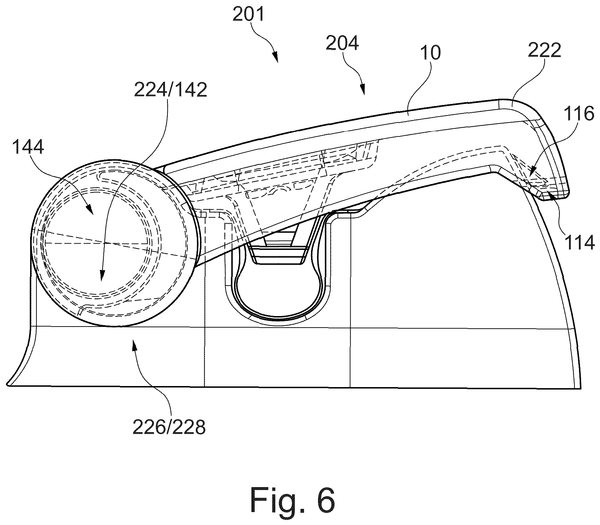

Figur 6 ist ein Gehäuse 201 gemäß einem dritten Ausführungsbeispiel der vorliegenden Offenbarung in einer durchscheinenden Seitenansicht.

Gemäß dem dritten Ausführungsbeispiel ermöglicht ein elastischer Abschnitt 222 des Deckels 204 die Ausweichbewegung des deckelseitigen Rastelements 114 gegenüber dem gehäuseseitigen Rastelement 116. Die gewünschte Elastizität wird durch ein geeignetes elastisches Design in Form einer Dünnwandigkeit zumindest des als Verrundung ausgestalteten Übergangsbereichs 222 des Deckels 204 bereitgestellt. Der Übergangsbereich 222 verbindet den flächigen Hauptabschnitt 10 des Deckels 204 mit dessen Rand, an dem das deckelseitige Rastelement 114 gebildet ist.

Damit hat das dritte Ausführungsbeispiel einen teilweise flexiblen Deckel 204, der über die Rastnase 116 "gezogen" werden kann. Durch eine Dünnwandigkeit der Rastkontur vorne (in Figur 6 rechts) wird die Elastizität vorne erreicht. Dabei wird die Rastfläche vorne (in Figur 6 rechts) im Deckel 204 durchgängig ausgeführt und von den Seitenflächen freigestellt.

Dadurch kann beim dritten Ausführungsbeispiel auf die Elastizität der Verbindungsabschnitte 134 und insbesondere auf die Aktivierungsschrägen 136, 138 in den Achsaufnahmen 128 des zweiten Ausführungsbeispiels gemäß den Figuren 4 und 5 verzichtet werden.

### Bezugszeichenliste:

- 1; 101; 201: Gehäuse
- 2; 102: Hauptkörper
- 4; 104; 204: Deckel
- 6: Blutschlauchaufnahme
- 7: Luftblasensensorik
- 8: Niederhalter
- 10: Hauptabschnitt
- 12: Schwenkachse
- 14; 114: deckelseitiges Rastelement
- 16; 116: hauptkörperseitiges Rastelement
- 18: Ausweichbewegung / Doppelpfeil
- 20: elastische Bewegung / Doppelpfeil
- 22: elastischer Abschnitt / Federzunge / Federlasche
- 24; 124; 224: Achszapfen
- 26; 126; 226: Schwenkgelenk
- 28; 128; 228: Achsaufnahme
- 30: Hülsenabschnitt
- 30a: gekrümmte Anlage
- 32: Abschlussdeckel
- 134: Verbindungsabschnitt
- 136: deckelseitige Aktivierungsschräge
- 138: hauptkörperseitige Aktivierungsschräge
- 140: Rippe
- 142: Stirnfläche
- 144: Einführschräge
- 222: elastischer Abschnitt / Übergangsbereich

## Patentansprüche

1. Gehäuse (1; 101; 201) eines Sicherheitsluftdetektors einer Dialysemaschine, wobei das Gehäuse (1; 101; 201) einen Hauptkörper (2; 102) mit einer Blutschlauchaufnahme (6) aufweist, wobei benachbart zur oder in der Blutschlauchaufnahme (6) eine Luftblasensensorik (7) gehäusefest angeordnet ist, wobei die Blutschlauchaufnahme (6) von einem Deckel (4; 104; 204) zumindest abschnittsweise abdeckbar ist, der mittels zweier Schwenkgelenke (26; 126; 226) um eine Schwenkachse (12) gegenüber dem Hauptkörper (2; 102) schwenkbar ist, wobei jedes Schwenkgelenk (26; 126; 226) einen hauptkörperseitigen Achszapfen (24; 124; 224) und eine deckelseitige Achsaufnahme (28; 128; 228) hat, wobei die Achsaufnahme (28; 128; 228) einstückig und integral mit einem Hauptabschnitt (10) des Deckels (4; 104; 204) gebildet ist, und wobei zwischen einem der Schwenkachse (12) gegenüberliegenden Rand des Hauptabschnitts (10) und dem Hauptkörper (2; 102) eine Rastvorrichtung mit zumindest einem hauptkörperseitigen Rastelement (16; 116) und mit zumindest einem deckelseitigen Rastelement (1;114) gebildet ist, **dadurch gekennzeichnet, dass** ein Ausweichen der beiden Rastelemente (14, 16; 114; 116) relativ zueinander durch eine Elastizität ermöglicht ist, die in mindestens einer der integral und einstückig mit oder an dem Deckel (104) gebildeten Achsaufnahmen (128) oder in mindestens einem integral und einstückig mit oder an dem Deckel (4; 204) gebildeten elastischen Abschnitt (22; 222) vorgesehen ist.

2. Gehäuse (1; 101; 201) nach Anspruch 1, **dadurch gekennzeichnet, dass** die Achsaufnahme (128) und/oder der elastische Abschnitt (22; 222) von einem Werkstoff gebildet ist, der elastischer und/oder weicher als der Werkstoff der übrigen Abschnitte des Deckels (4; 104; 204) ist.

3. Gehäuse (1; 101; 201) nach einem der vorhergehenden Ansprüche, **dadurch gekennzeichnet, dass** die Achsaufnahme (28; 128; 228) an einer der Rastvorrichtung zugewandten Seite eine gekrümmte Anlage (30a) hat, die ein Gleitlager für den Achszapfen (24; 124; 224) bildet.

4. Gehäuse (1; 101; 201) nach Anspruch 3, **dadurch gekennzeichnet, dass** die Achsaufnahme (28; 128; 228) einen um den Achszapfen (24; 124; 224) umlaufenden Hülsenabschnitt (30) hat, wobei die gekrümmte Anlage (30a) ein Abschnitt des Hülsenabschnitts (30) ist.

5. Gehäuse (1; 101) nach Anspruch 4, **dadurch gekennzeichnet, dass** der Hülsenabschnitt (30) oval ist.

6. Gehäuse (1; 101) nach Anspruch 4 oder 5, **dadurch gekennzeichnet, dass** der Hülsenabschnitt (30) von einem stirnseitigen Abschlussdeckel (32) abgedeckt ist, womit die Achsaufnahme (28; 128) topfartig ist.

7. Gehäuse (1) nach einem der vorhergehenden Ansprüche, **dadurch gekennzeichnet, dass** der elastische Abschnitt (22) an der Achsaufnahme (28) gebildet ist und radial gegen einen Außenmantel des Achszapfens (24) gespannt ist, wobei der elastische Abschnitt (22) in einem von der Rastvorrichtung abgewandten Umfangsbereich des Außenmantels an diesem anliegt.

8. Gehäuse (1) nach einem der vorhergehenden Ansprüche, **dadurch gekennzeichnet, dass** der elastische Abschnitt von einer Federzunge (22) gebildet ist, die im Innern der Achsaufnahme (28) befestigt ist und sich zumindest abschnittsweise tangential zum Achszapfen (24) erstreckt.

9. Gehäuse (1) nach Anspruch 8, **dadurch gekennzeichnet, dass** die Federzunge (22) an ihrer dem Achszapfen (24) zugewandten Seite eine konkave Krümmung aufweist, in die der Achszapfen (24) eintaucht.

10. Gehäuse (101) nach einem der Ansprüche 1 bis 6, **dadurch gekennzeichnet, dass** das Ausweichen der beiden Rastelemente (114, 116) relativ zueinander durch die Elastizität der beiden Achsaufnahmen (128) ermöglicht ist.

11. Gehäuse (101) nach einem der Ansprüche 1 bis 6, **dadurch gekennzeichnet, dass** jede Achsaufnahme (128) über einen Verbindungsabschnitt (134) mit dem Hauptabschnitt (10) des Deckels (104) einstückig und integral verbunden ist, **dadurch gekennzeichnet, dass** das Ausweichen der beiden Rastelemente (114, 116) relativ zueinander durch die Elastizität der beiden Verbindungabschnitte (134) ermöglicht ist.

12. Gehäuse (101) nach Anspruch 10 oder 11, **dadurch gekennzeichnet, dass** im Innern der Achsaufnahme (128) beabstandet zur Schwenkachse (12) eine deckelseitige Aktivierungsschräge (136) gebildet ist, und dass am Achszapfen (124) beabstandet zur Schwenkachse (12) eine hauptkörperseitige Aktivierungsschräge (138) gebildet ist, wobei beide Aktivierungsschrägen (136, 138) gemeinsam an einer von der Rastvorrichtung abgewandten Seite der Schwenkachse (12) angeordnet sind, und wobei beide Aktivierungsschrägen (136, 138) in gleicher Richtung und vorzugsweise mit etwa gleichem Winkel schräg zur Schwenkachse (12) angeordnet sind, und wobei bei dem Ausweichen der Rastelemente (114, 116) die beiden Aktivierungsschrägen (136, 138) die beiden Achsaufnahmen (128) entlang der Schwenkachse (12) auseinanderdrängen.

13. Gehäuse (101) nach Anspruch 12, **dadurch gekennzeichnet, dass** der Achszapfen (24) eine Stirnfläche (142) hat, wobei die hauptkörperseitige Aktivierungsschräge (138) ein von der Rastvorrichtung abgewandter Oberflächenabschnitt der Stirnfläche (142) ist.

14. Gehäuse (101) nach Anspruch 13, **gekennzeichnet durch** eine Einführschräge (144), die schräg zur Schwenkachse (12) und schräg zur hauptkörperseitigen Aktivierungsschräge (138) angeordnet ist, und die ein Oberflächenabschnitt der Stirnfläche (142) ist.

15. Gehäuse (101) nach Anspruch 13 oder 14, **dadurch gekennzeichnet, dass** im Innern der Achsaufnahme (128) ein Vorsprung oder eine Rippe (140) gebildet ist, der oder die an einer von der Rastvorrichtung abgewandten Seite der Schwenkachse (12) angeordnet ist, und der oder die sich radial zur Schwenkachse (12) nach innen erstreckt, wobei die deckelseitige Aktivierungsschräge (136) an dem Vorsprung oder an der Rippe (140) gebildet ist.

16. Gehäuse (201) nach einem der Ansprüche 1 bis 4, **dadurch gekennzeichnet, dass** der elastische Abschnitt (222) im Deckel (204) zwischen dem Hauptabschnitt (10) und dem Rand gebildet ist.
